# EUROPEAN PATENT APPLICATION

(11) **EP 3 895 667 A1**
(43) Date of publication of application: **20.10.2021**
(21) Application number: 21460020.7
(22) Date of filing: 12.04.2021
(51) Int. Cl.: A61F 2/64, A61F 2/74, A61F 2/50

(54) **KNEE PROSTHESIS**

(30) Priority: 17.04.2020 PL 43359820
(71) Applicant: CONTUR 2000 s.c. Piotr Trzcinski Grzegorz Wieckowski, 59-800 Luban (PL)
(72) Inventor: Wesolowski, Pawel, 59-900 Zgorzelec (PL); Trzcinski, Piotr, 59-730 Nowogrodziec (PL); Wieckowski, Grzegorz, 59-800 Luban (PL)

(57) **Abstract**

The subject of the invention is a knee prosthesis.

The knee prosthesis mounted between the upper body equipped with the upper mounting adapter and the body equipped with the lower mounting adapter and equipped with a shock absorber, characterized by the fact that in the body (7) on the shock absorber piston rod (19) a roller (8) is mounted in the roller guiding element (9), and above the roller (8) there is a cam of the deceleration mechanism (6) fixed in the upper body (2).

## Description

The subject of the invention is a knee prosthesis.

The articulated mechanism, known from the Polish patent specification as No. PL219055, consists of a first link connected to the second link and a drive unit. The first link consists of a mandrel and a head mounted thereon on which the carriage of the second member bases. The first connector and a second rocker arm are rotatably attached to the head, which connect the head to the trolley. The first rod, on the other hand, is part of a driving assembly that includes an actuator attached to the shaft of the second link.

The prosthetic knee joint known from European Patent Specification No. EP2316390 has a swing phase control system with a piston which is connected to the femoral connector and movable with respect to the cylinder, and a cylinder connected to the lower leg connector, the cylinder being pivoted about at least one axis with respect to the lower leg which is remote from the axle via an articulation point. The cylinder is connected to an elastic substance that rests on the lower leg connector.

The knee joint of the prosthesis of the leg with a braking device known from the German patent description No. DE19506426, consists of the lower part of the joint, the upper part of the joint, connected to one of the joint parts in a manner protected against rotation of the joint axis, forming the central part of the joint, a rocker lever, the projecting end of which is attached to an axis of rotation which is parallel and forward to the axis of articulation and which embraces the pivot at its bent end, provided with a braking device controlled by the load on the leg. The central part of the joint is provided with a set of two circumferential displacement chambers filled with hydraulic oil and surrounding at least part of its circumference the axis of the joint. These chambers are mutually separated by a rotary piston mounted on the axis of the joint and interconnected by an oil pipe. The central part is also provided with a sliding valve piston, cooperating on one side with a spring holding it in the open position and on the other side with an adjusting device located in the lower part of the joint which closes the valve when the joint is bent.

The joint of the prosthesis with a braking device known from the European patent specification as a No. EP0739615, is a knee joint of a prosthesis of a leg with a braking device, composed of an upper joint part, a lower joint part and a joint axis, which is the axis of the braking device, pivotally connecting both joint parts and connected secured against rotation with the lower part (of the joint, mounted in the brake sleeve, secured against rotation with the clamping element, on which the upper part of the joint is articulated by the oscillating axle in such a way that the upper part of the joint, when loaded, presses against the element clamping, and thus onto the brake sleeve, transmitting the braking action to the pivot axle).

The joint for prostheses of the lower limbs, known from the Polish utility model No. RU060154, has the form of mutually perpendicularly positioned two sleeves, i.e. the upper and lower ones, mounted on pins, fixed in fork handles, constituting a biaxial joint. The axes of rotation of these sleeves are spaced apart by a magnitude, and the holders are further provided with upper and lower plates. The upper plate of the upper handle is permanently connected to the upper threaded connector, and the bottom plate of the lower handle is permanently connected to the lower threaded connector. The sleeves are mounted on pins movable in the range of 120 to 180°.

Articulated knee prosthesis known from European patent specification as a No. EP1849439, in which the prosthetic knee joint has four axial screws. Two opposing joint elements running obliquely are connected to a prosthetic shaft on one side and connected by a prosthetic foot on the other side. The other two articular elements pivot with each other as elongated articulation elements in an upright and a biasing position. The articular element axial bolt rotates and defines an eccentric element connected to the foot prosthesis, by rotation of which the distance between the two axle bolts changes.

The artificial limb connection with a square connector known from US Patent No. US6752835 has four axial pins, where four axial pins connect the knee head, two connecting plates, a fork and a seat to the lower leg together and form a triangular support. In addition, the pressure center is at the end of the inverted triangle formed by the four axial pins.

The braked knee joint known from US Patent No. US4685927 has a thigh part and a lower leg part which are provided with braking surfaces adapted to each other and which, due to the possibility of axial movement of the thigh part relative to the lower leg part, are pressed against each other during loading. The braking surfaces generally have a contour which is circular in vertical section and which has a constant radius with respect to the axis of rotation. The joint includes a suitable stop which ensures a jerk-free termination of the movement of the extension of the knee joint from the flexed position. The limiter is formed by providing a braking surface of the thigh part with a front end that is at a distance that increases from the axis of rotation and greater than the constant radius, and by providing a corresponding front end part of the braking surface of the lower leg so that as the elongation increases in an unloaded state of the knee joint, the constantly increasing area of the braking surfaces comes into frictional contact, which causes a stop.

The essence of the knee prosthesis according to the invention consists in the fact that in the body, on the shock absorber piston rod, a roller is mounted in the roller guiding element, and above the roller there is a cam of the deceleration mechanism fixed in the upper body.

Preferably, the roller guiding element is embedded in the guide sleeve mounted in the guide body, while the shock absorber piston is embedded in the hydraulic shock absorber mounted in the guide body through the mounting sleeve, the hydraulic adjustable shock absorber is attached to the guide body, at the same time the hydraulic adjustable shock absorber is connected through the shock absorber adjusting element, the actuating mechanism guide and the hydraulic valve body with the lower body, and the mounting adapter is connected with the hydraulic valve head, moreover, a spring is mounted on the hydraulic adjustable shock absorber, the hydraulic valve body and the hydraulic valve head constitute the hydraulic shock absorber actuating mechanism.

Preferably, a first axis of rotation with a bearing and a third axis of rotation with a bearing are mounted in the upper body, while the first end of the cable of the four-axis knee joint mechanism is rotatably mounted on the first axis of rotation with a bearing, the second end of which is rotatably mounted on the second axis of rotation with a bearing, mounted in the body, while the first end of the deceleration mechanism cam is mounted on the third axis of rotation with the bearing, and the other end of the deceleration mechanism cam is located in the body above the roller mounted in the element guiding the roller relative to the body.

The prosthetic's feature is that when it is weighted by the user, the mechanism for slowing down the bending movement of the knee is active and makes it easier for the user to descend stairs in particular. The mechanism is adjustable and the user can adjust the damping force (slowing down the bending movement) to his weight and preferences. In a situation when the prosthesis is not weighted, the mechanism that slows down the bending of the knee is turned off and the prosthesis can bend freely without additional resistance, which makes it much easier to swing the leg while moving it forward in the air - the "swing" movement. This mechanism can be used in multi-axis knee prostheses as well as in uniaxial knee prostheses. It can be a component of an active prosthesis (having its own drive) or a part of a passive knee prosthesis (without a drive).

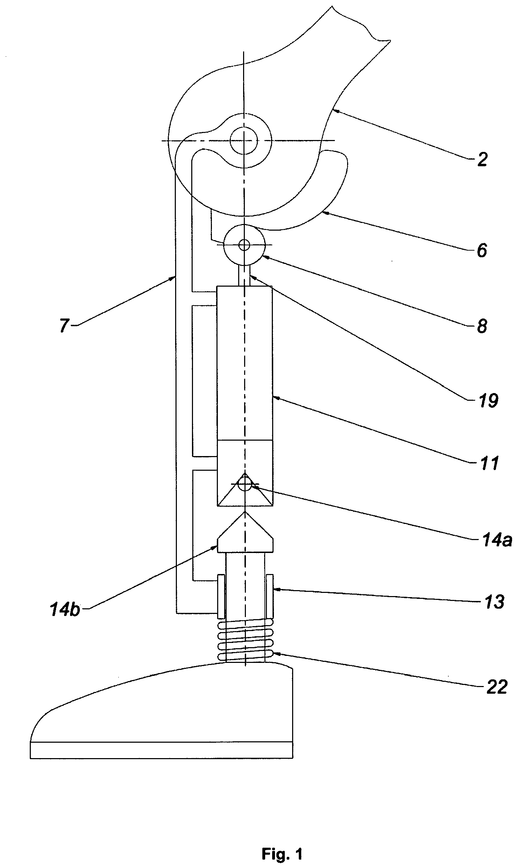

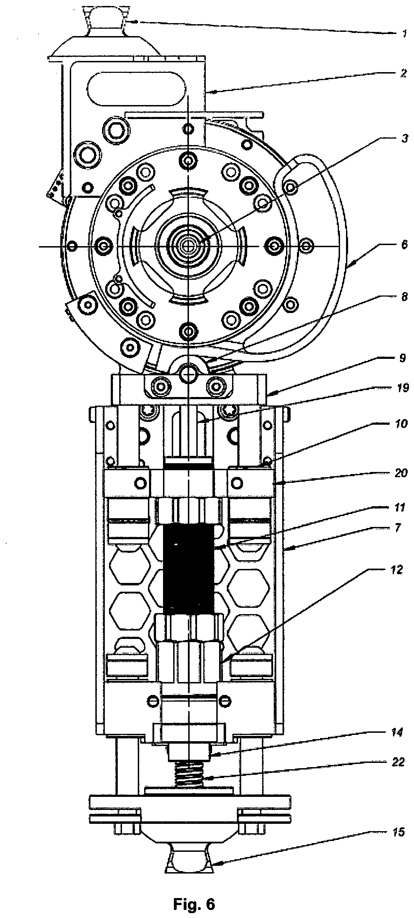

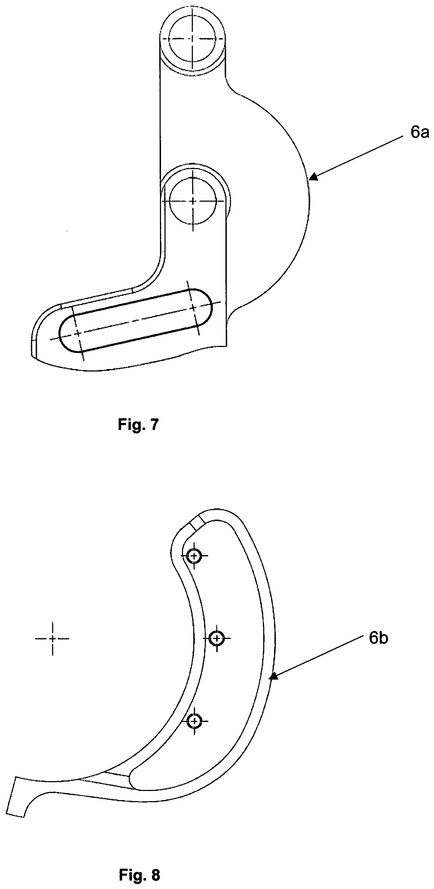

The subject of the invention in an exemplary embodiment is shown in the drawing, in which figure 1 shows a simplified diagram of a knee prosthesis with a hydraulic shock absorber activation mechanism, figure 2 - an erected four-axis knee prosthesis loosely changing the angular deflection value with the hydraulic shock absorber activation mechanism not activated, figure 3 - bent four-axis knee prosthesis loosely changing the value of the angular deflection. with the hydraulic shock absorber activation mechanism not activated, figure 4 - straightened four-axis knee prosthesis loosely changing the angular deflection value with the hydraulic shock absorber activation mechanism activated, figure 5 - bent four-axis knee prosthesis loosely changing the angular deflection value, with the hydraulic shock absorber activation mechanism activated, figure 6 - a uniaxial knee prosthesis with a hydraulic shock absorber engagement mechanism, figure 7 - a cam of a four-axis knee joint mechanism, and figure 8 - a cam of a uniaxial knee joint mechanism.

### Example 1

The knee prosthesis has a body 7 articulated with the upper body 2, in which a cam of the deceleration mechanism 6 is mounted, mounted over a roller 8, on the piston rod of a shock absorber 19 of a hydraulic adjustable shock absorber 11, which is equipped with a mechanism for activating a hydraulic shock absorber 14 constituting a valve consisting of a valve body hydraulic valve 14a and the hydraulic valve head, 14b. The head of the hydraulic valve 14b is mounted on the guide of the switching mechanism 13 equipped with a spring 22.

### Example 2

The knee prosthesis, which is a four-axis knee prosthesis, has an upper mounting adapter 1 mounted on the upper body 2, in which the first axis of rotation with a bearing 3 and a third axis of rotation with a bearing 16 are mounted, while the first end is rotatably mounted on the first axis of rotation with a bearing 3, the tie rod of the four-axis mechanism of the knee joint 4, the second end of which is rotatably mounted on the second axis of rotation with bearing 5 mounted in the body 7, while the first end of the cam of the four-axis deceleration mechanism 6a is mounted on the third axis of rotation with bearing 16, also on the fourth axis of rotation with bearing 17 in the body 7. The other end of the cam of the four-axis deceleration mechanism 6a is located in the body above the roller 8 mounted in the roller guiding element 9 relative to the lower body 7, the roller guiding element 9 being mounted on the piston rod of the damper 19 provided with the guide body 20. The guide element roll 9 sediments is located in the guide bushing 10 installed in the guide body 20. The shock absorber piston 19 is mounted by a hydraulic adjustable shock absorber 11, shock absorber adjusting element 12, a guide for the actuation mechanism 13 and the actuation mechanism of the hydraulic shock absorber 14 with a lower mounting adapter 15 mounted on the body 7, also hydraulic The adjustable shock absorber 11 is attached to the guide body 20 by means of a mounting sleeve 21, and a spring 22 is mounted on the hydraulic adjustable shock absorber 11. The actuating mechanism of the hydraulic shock absorber 14 has a hydraulic valve body 14a and a hydraulic valve head 14b made in the form of a valve.

### Example 3

The knee prosthesis is made as in the second example, with the difference that the axis of rotation with bearing 17 is equipped with a brake 18.

### Example 4

The knee prosthesis is made as in the first and second examples, with the difference that it is a uniaxial knee prosthesis equipped with a cam of the uniaxial mechanism of the knee joint 6b. A prosthesis with a mechanism for engaging a hydraulic damper 14 that slows down the bending movement of the knee and a system that activates this mechanism only when the prosthesis is loaded by the user.

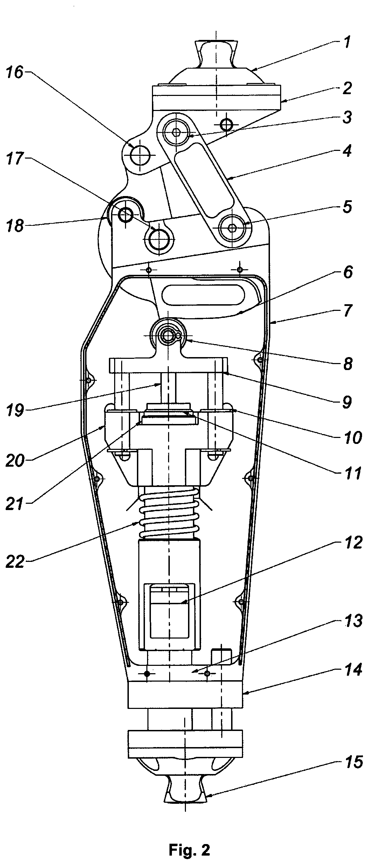

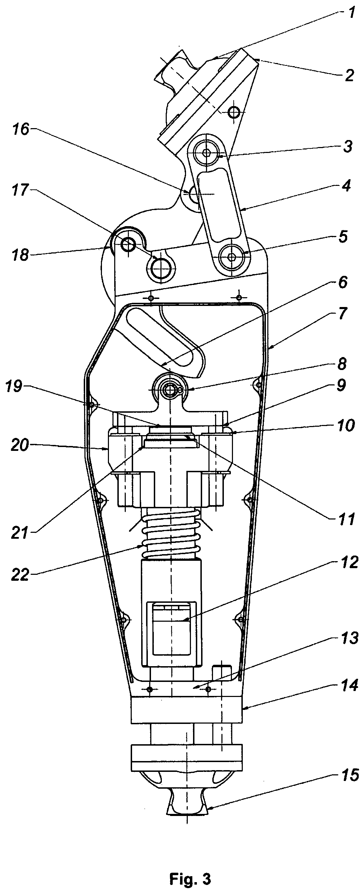

The four-axis knee prosthesis (figure 2, figure 3) is equipped with a mechanism for slowing down the flexion movement. This mechanism consists of a cam of the four-axis mechanism of the knee joint 6a, a roller 8 cooperating with it mounted on the roller guide 9, a hydraulic adjustable shock absorber 11 to dissipate energy, a shock absorber adjusting element 12 for adjusting the force of slowing the bending movement of the knee prosthesis, and a system activating the shock-actuation mechanism an energy-dissipating hydraulic 14 which only works when the prosthesis is weighted. The knee joint prosthesis, unloaded by the user, successively in an upright and partially bent position, with the hydraulic shock absorber 14 not activated, slows down the bending movement and loosely changes the angular deflection value without much resistance.

The actuation mechanism of the hydraulic damper 14 to slow the knee bending movement is inactive, which allows the knee joint to be flexed very easily while moving the leg forward in the air. The actuation mechanism of the hydraulic shock absorber 14 is moved away from the body 7, which results in the fact that the adjustable hydraulic shock absorber 11 equipped with the body of the hydraulic valve 14a and the head of the hydraulic valve 14b is completely open and does not introduce resistance during bending of the prosthesis. The knee flexion slowing system is disabled and the user can flexibly flex the knee prosthesis.

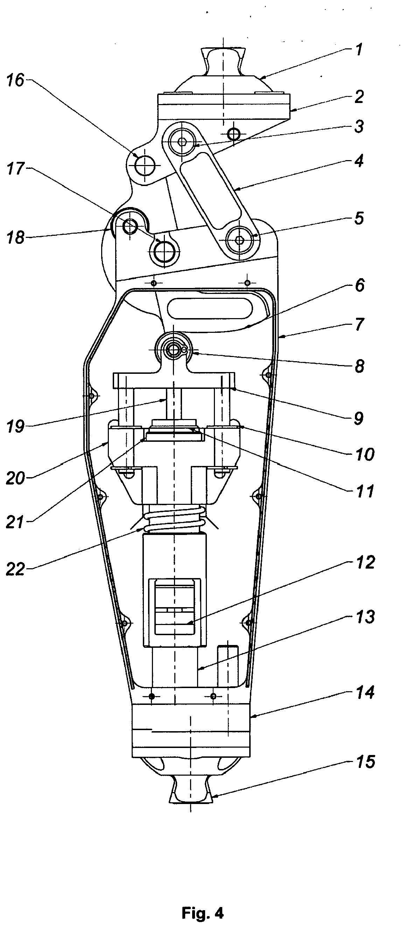

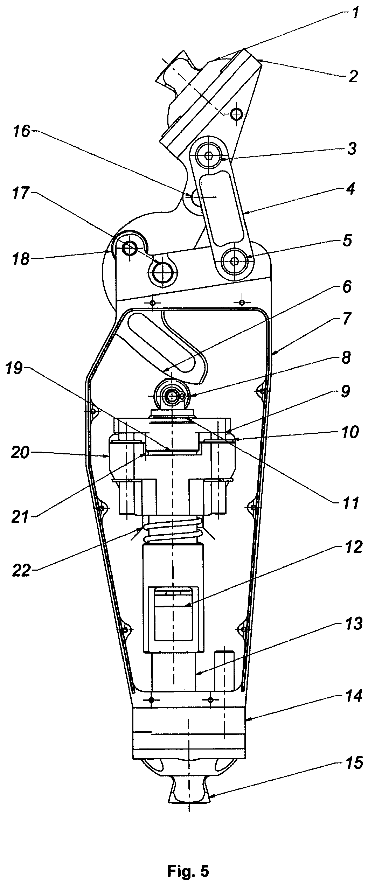

The knee joint prosthesis subjected to a user load (figure 4, figure 5) has an active mechanism for slowing down the knee flexion movement and in this situation the knee flexion movement is slowed down, which is especially useful when going down stairs.

The actuation mechanism of the hydraulic shock absorber 14 is pushed against the body 7, which results in the fact that the adjustable hydraulic shock absorber 11 equipped with a hydraulic valve body 14a and a hydraulic valve head 14b is closed and introduces hydraulic resistance during bending of the prosthesis. The value of this resistance is regulated and matched to the user so that the bending speed of the knee prosthesis is correct. The value of this speed depends on the shape of the cam of the four-axis mechanism of the knee joint 6a and the set resistance force resulting from pressing the piston rod 19 of the adjustable hydraulic shock absorber 11.

### List of symbols in the drawing:

1. The mounting adapter,
2. The upper body,
3. The first axis of rotation no. with bearing,
4. The tendon of the four-axis mechanism of the knee joint,
5. The second axis of rotation with bearings,
6. The deceleration mechanism cam,
6a. The cam of the four-axis mechanism of the knee joint,
6b. The cam of the uniaxial mechanism of the knee joint,
7. The body,
8. The roll,
9. The roller guiding element,
10. The guide sleeve,
11. The hydraulic adjustable shock absorber,
12. The shock absorber adjusting element,
13. The switching mechanism guide,
14. The hydraulic shock absorber activation mechanism,
14a. The hydraulic valve body,
14b. The hydraulic valve head,
15. The bottom mounting adapter,
16. The third axis of rotation with bearings,
17. The fourth axis of rotation with bearings,
18. The brake,
19. The shock absorber piston rod,
20. The guide body,
21. The fixing sleeve,
22. The spring.

## Claims

1. The knee prosthesis is mounted between the upper body equipped with an upper mounting adapter and the body equipped with the lower mounting adapter and equipped with a shock absorber, **characterized in that** in the body (7) on the shock absorber piston (19) a roller (8) is mounted in the roller guiding element (9), and above the roller (8) there is a cam of the deceleration mechanism (6) fixed in the upper body (2).

2. The prosthesis according to claim 1, is **characterized in that** the roller guiding element (9) is embedded in the guide bushing (10) mounted in the guide body (20), while the shock absorber piston (19) is embedded in the hydraulic shock absorber (11) mounted in the guide body (20) by the mounting sleeve (21), the hydraulic adjustable shock absorber (11) is attached to the guide body (20) at the same time, the hydraulic adjustable shock absorber (11) is connected through the shock absorber adjusting element (12), the switching mechanism guide (13) and the hydraulic valve body (14a) with the lower body (7), while the mounting adapter (15) is connected to the head of the hydraulic valve (14b), moreover, a spring (22) is mounted on the adjustable hydraulic shock absorber (11), the body of the hydraulic valve (14a) and the head of the hydraulic valve (14b) are the actuation mechanism of the hydraulic shock absorber (14).

3. The prosthesis according to claim 1, is **characterized in that** the first axis of rotation with a bearing (3) and a third axis of rotation with a bearing (16) are mounted in the upper body (2), and the first end of the linkage of the mechanism is rotatably mounted on the first axis of rotation with a bearing (3), a four-axis knee joint (4), the second end of which is rotatably mounted on the second axis of rotation with a bearing (5) mounted in the body (7), while the first end of the deceleration mechanism cam (6) is mounted on the third axis of rotation with a bearing (16), and the other end of the cam of the deceleration mechanism (6) is located in the body (7) above the roller (8) mounted in the roller guide (9) with respect to the body (7).
